# EUROPEAN PATENT SPECIFICATION

(11) **EP 3 320 465 B1**
(45) Date of publication and mention of the grant of the patent: **01.05.2019**
(21) Application number: 16744322.5
(22) Date of filing: 07.07.2016
(51) Int. Cl.: G16H 40/40

(54) **METHOD FOR MONITORING A MEDICAL DEVICE**
VERFAHREN ZUR ÜBERWACHUNG EINER MEDIZINISCHEN VORRICHTUNG
PROCÉDÉ PERMETTANT DE SURVEILLER UN DISPOSITIF MÉDICAL

(30) Priority: 07.07.2015 SE 1550988
(43) Date of publication of application: 16.05.2018
(73) Proprietor: Brighter AB (Publ), 164 32 Kista (SE)
(72) Inventor: SJÖSTEDT, Truls, 187 76 Täby (SE)
(74) Representative: Noréns Patentbyrå AB
(86) International application number: PCT/EP2016/066154
(87) International publication number: WO 2017/005861

(56) References cited:
- WO-A2-2006/083876
- WO-A2-2008/067314
- US-A1- 2004 117 204
- US-A1- 2008 244 437
- US-A1- 2015 106 020

## Description

### FIELD OF THE INVENTION

This invention relates to a method and a system for monitoring the health of a portable medical device, by for example, determining if the medical device needs to be replaced or serviced.

### BACKGROUND

The number of injectable medicaments that are self-administered by patients is constantly growing. Insulin is perhaps the most well-known example of a medicament that the patient injects himself, but in addition also antibodies, growth hormone, EPO, and certain chemotherapy drugs are self-administered by the patient by injection.

Injection is most frequently done with a handheld injection device, which typically is used from one to three times per day (as opposed to an infusion device that continuously deliver a medicament).

It is of great importance, for patient safety reasons, that injection devices work in a correct and dependable manner. At the same time such devices, which usually are carried by the users at all times, may be exposed to extreme temperatures or humidity, or shock (if the user drops the device), which may damage the device. Furthermore, daily use causes wear of the device.

Such medical devices may malfunction from time to time, and they may then need service or replacement.

US2006/0173417 discloses a medical device for injection of insulin that can transmit the amount of injected insulin to a remote computer. However, the system described therein does not detect if the device needs service or replacement.

### SUMMARY OF INVENTION

In a first aspect of the invention there is provided a method comprising the steps of a) providing a portable medical device for the injection of a medicament comprising means for injection of a medicament, a cellular radio transceiver and at least one sensor, said sensor being selected from the group consisting of a temperature sensor, a humidity sensor, an accelerometer and an operations counter, the portable medical device using the sensor to determine a value of a parameter that is sensed by the sensor, b) the portable medical device using the cellular radio transceiver to establish a data connection to a remote computer with the use of a cellular network, c) the portable medical device using the data connection to transfer the value from step a) to the remote computer and d) the remote computer using the value from step c) to determine whether the portable medical device is in need of scrapping, service or replacement.

Individual patients may be reluctant or may forget to report a malfunctioning device. It is therefore an advantage that the operation of medical devices can be monitored remotely, so that a health provider can take steps so that the medical device is serviced or replaced.

One advantage of the invention is that sensor values are reported to and analysed by a remote computer. The remote computer determines whether the medical device should, for example, be scrapped, serviced or replaced. For example, if a threshold value for temperature is used for the decision, a threshold value is stored in the remote computer. From time to time the threshold value may have to be adjusted. Such adjustments may be triggered by new findings regarding failures of medical devices. For example, it is discovered that a medical devices is more sensitive to temperature or humidity than previously thought. The invention provides convenient way to adjust such threshold values.

If the threshold value is stored in the device a change in a threshold value for a certain model of medical device must be done in a every single device of that model. This may involve updating the software in a large number of devices, which is cumbersome.

Updating of software for a medical device also involves safety issues. For example, there is a risk that the software is sabotaged when a software update is distributed to a number of devices. This may put the patients at risk. According to the invention, the threshold value can be conveniently adjusted at one place (the remote computer), which is safe and simple and reduces the risk for sabotage.

Also, gathering data from the sensors in a central remote computer creates a large data set from a plurality of medical devices. The data set can be used to build knowledge about usage patterns and sensor data in its relation to malfunctioning of devices. The data set may be used to improve decision rules regarding service or replacement of the medical device. Machine learning or data mining technologies may be used for this purpose. Therefore the method can be used as a research tool to discover new conditions that may cause a medical device to malfunction.

The method provides a convenient way for a health care provider to monitor a fleet of medical devices.

The sensor of the medical device may be an operations counter. The value sensed by the operations counter may be proportional to the amount of medicament that is injected by the medical device. The transferred value may be a value from an operations counter, and the method may then comprise the step of adding the determined value from step a) to a total operations value that is previously stored in the remote computer or in the medical device, to obtain an updated total operations value, and where the updated total operations value is used in step d).

In one embodiment the sensor is a temperature sensor.

In one embodiment the sensor is a humidity sensor.

In a preferred embodiment the sensor is an operations counter. In an even more preferred embodiment the medical device has a sensor which is an operations counter and at least one sensor selected from the group consisting of a temperature sensor, a humidity sensor and an acceleration sensor.

The method may comprise the step of determining the geographical position of the portable medical device, and where information about the geographical position is transferred to the remote computer. The geographical position may be determined by using mobile phone tracking using the cellular radio transceiver of the medical device. The geographical position may also be determined with the use of a mobile phone that is carried by the user of the medical device.

Step d) of the method comprise the use of a decision rule. Step d) of the method may comprise the step of the remote computer establishing if the value exceeds a threshold value. This is simple and convenient way of monitoring a medical device.

Step d) of the method may comprise the step of the remote computer applying a decision rule, said decision rule being generated by applying machine learning to a data set, said data set comprising data previously collected from comparable portable medical devices, said data set also comprising data about previous failure or error codes of such similar portable medical devices.

The data set may comprise information about values from sensors previously collected according to steps a) to c) of the method described above. The data set may comprise information about the geographical position of the portable medical device.

The geographical position of the portable medical device for which the decision rule is applied can be used together with a time point to retrieve data for at least one additional parameter, which additional data is used to make the determination in step d). The data for the additional parameter may be added to the data set, for improvement of the data set and the decision rule (generally the decision rule improves as the data set grows). The additional parameter can be a parameter selected from the group consisting of temperature, humidity, weather, calendar data, air pollution, air pressure, and road traffic information.

The data set may comprise data for an additional parameter for the medical device from the group consisting of temperature, humidity, acceleration or time in free fall, weather, calendar data, air pollution, air pressure, geographical position, time points, number of operations, amount of medicament injected, time of injection, and geographical position at time of injection. The data for these parameters may be added to the dataset as described above, in order to increase the size of the dataset. Preferably there is a large number of data points in the dataset. Statistical methods can be used to decide which kind of data that are allowed to contribute to a decision rule.

The dataset may comprise information about the previous behaviour of users of medical devices from a social network service, and the remote computer in step d) uses information about the behaviour from a social network service regarding the user of the portable device for which the decision rule is applied. The dataset may thus also comprise information made available by users of comparative medical devices, said information being from a social network service, and the remote computer in step d) uses information regarding the user of the portable device for which the decision rule is applied, said information made available by the user to the social network service. Such information from a social network service may include data about the behaviour of the user, for example if the user does sports, if the user checks in to certain locations, time of posting information, etc.

The method is preferably carried out by a processing unit in the medical device and by the remote computer.

In a second aspect of the invention it is provided a system for deciding if a portable medical device for the injection of a medicament should be serviced or replaced, said system comprising a portable medical device and a remote computer, said portable medical device comprising injections means and a processing unit, a memory and a cellular radio transceiver and at least one sensor selected from the group consisting of a temperature sensor, a humidity sensor, an accelerometer, and an operations counter, said portable medical device being configured to store data from the at least one sensor in the memory, said remote computer and portable medical device adapted to establish a data connection with the use of a cellular network, the portable medical device being configured to use the data connection to transfer the sensor data to the remote computer, the remote computer configured to use the sensor data to determine whether the portable medical device is in need of scrapping, service or replacement.

The remote computer of the system may have decision rule software, said decision rule software being generated by applying machine learning to a data set, said data set comprising sensor data previously collected from comparable portable medical devices, said data set further comprising data about any previous failures of such comparable portable medical devices.

In a third aspect of the invention there is provided a portable medical device for the injection of a medicament, said medical device comprising means for injection of a medicament, said medical device further comprising a cellular radio transceiver and at least one sensor, said sensor selected from the group consisting of a temperature sensor, a humidity sensor, an accelerometer, and an operations counter, the portable medical device further comprising a processing unit, said device being configured to use the sensor to determine a value for a parameter that is sensed by the sensor, to use the cellular radio transceiver to establish a data connection to a remote computer with the use of a cellular network and to use the data connection to transfer the determined value to the remote computer.

The device may be configured to, after transfer of the determined value to the remote computer, to receive from the remote computer a signal causing the medical device to notify a user of the medical device that the device needs service or replacement, or shutting down the medical device.

In one embodiment at least one sensor is a temperature sensor or a humidity sensor.

The device may have an activation switch, whereby a user can cause the humidity or temperature sensor to measure temperature or humidity and store the temperature value or humidity value in the memory of the medical device. The activation may switch on the medical device or wake it up from sleep. The device can be configured to, when the user has used the activation switch, to wait for further user action during a predetermined waiting period, and, if the user takes further action within the waiting period, establish a data connection to the remote computer and transfer the determined value to the remote computer, and if the user does not take further action within the waiting period, being configured to transfer the determined value the next time a data connection is established with the remote computer. The medical device may be configured to switch off or go to sleep if the user does not take any action within the waiting period. This has the advantage of saving battery power.

In a fourth aspect of the invention there is provided a remote computer being configured to receive from a portable medical device for the injection of a medicament, using a data connection, a value for a parameter selected from the group consisting of temperature, humidity, acceleration, number of injections carried out or amount of medicament injected, and using the value to determine whether the portable medical device is in need of scrapping, service or replacement. The remote computer may, when determining if the portable medical device is in need of scrapping service or replacement apply a decision rule, said decision rule being generated by applying machine learning to a data set, said data set comprising sensor data previously collected from comparable portable medical devices, said data set also comprising data about any previous failures of the comparable portable medical devices.

### BRIEF DESCRIPTION OF DRAWINGS

The invention will now be described in more detail by means of examples and with reference to the accompanying figures in which:
Fig. 1 is a schematic overview of a system, which shows both hardware and software components.
Fig. 2 is a schematic overview of hardware and/or software components in a remote computer.
Fig. 3 shows how an operations counter can be arranged.
Fig. 4 is a flowchart that shows a method.
Fig. 5 is a flowchart that shows a method that involves a threshold.
Fig. 6 is a flowchart that shows a method that involves machine learning.
Fig. 7 is a flowchart that shows a method for switching on a sensor and transfer of sensor data.

Although it is referred to "injection", "time of injection", "injection means" "injection device", etc. in this application it should be understood that an injection also refers to cases when medicament is ejected by the medical device without being injected into a patient. That may be the case for example, when a user primes a syringe by ejecting a small amount of medicament for the purpose of checking that the device works as intended. Thus, as used herein, the term "injection" means that an "ejection" also is carried out by the medical device.

The disclosed threshold values, for example 50°C, are provided as examples only and are not limiting in any way. The actual threshold values to be used when using the invention can be found by carrying out tests, found in specifications for components used in the actual device or by machine learning as described below. The thresholds are preferably predetermined meaning that the threshold is fixed at least before the remote computer makes the determination step.

### DETAILED DESCRIPTION

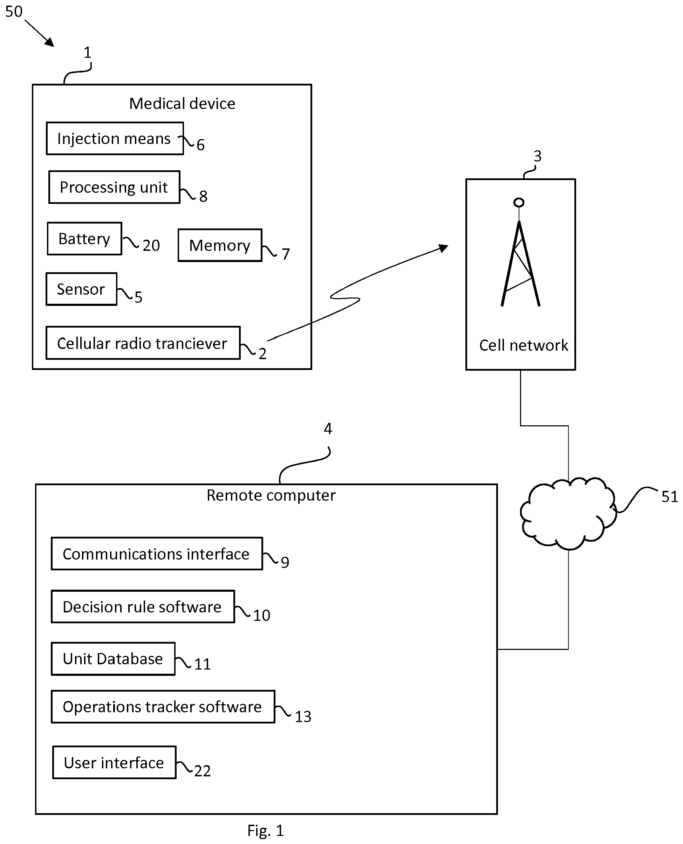

With reference to Fig. 1, the portable medical device 1, hereafter referred to as the medical device 1, is portable. It is intended to be carried by the user, for example in a pocket or in a hand bag. The medical device 1 is preferably a handheld medical device.

The medical device 1 is a device for injection of a medicament such as growth hormone, antibodies (such as antibody-based medicaments) or insulin. Typically the medical device 1 is used by a person to inject himself or herself from one to three times per day. This is the most common way for a person to administer insulin to himself or herself. Thus the medical device is not a device for infusion, that is, continuous delivery of medicament.

The medical device 1 t has injection means 6. The injections means 6 may be arranged as is known in the art of injection devices. The injection means 6 may comprise a site for attaching a replaceable cartridge that contains a medicament. The cartridge typically has a site for attaching an injection syringe. The injection means 6 also typically comprises a plunger for creating a pressure in the cartridge and a dose setting means that controls how far the plunger will travel. The plunger is powered by a drive mechanism. The injection means 6 also typically comprises an injection actuator, for example a button that can be pressed by the user. This causes movement of the plunger to the extent allowed by the dose setting means. An example of suitable injections means can be found in WO2015076745 and references cited therein.

The medical device 1 has a cellular radio transceiver 2 for communication with a cell network 3 that allows data traffic. The cell network 3 may be for example a GPRS, UMTS, 3G, 4G, LTE or similar cell network. The cell radio transceiver 2 typically comprises a SIM card - or a site for attaching a SIM card - for identification of the medical device 1 in the cell network 3. The cellular radio transceiver 2 is capable of establishing a data connection between the medical device 1 and a remote computer 4, for example via the internet 51. However, voice traffic is not necessary and is not necessarily enabled by cell radio transceiver 2. An advantage with communication over a cellular network over, for example, Wi-Fi, is that cell coverage does not require a manual log in procedure and is available almost everywhere. However, communication between medical device 1 and remote computer 4 may, in addition, involve other types of network of connection types that supports data communication for example non-wireless technologies, such as wire-bound technologies such as Ethernet.

The medical device 1 has at least one sensor 5. The numbers of sensor may be one, two, three, four, or more. The medical device 1 also has at least one memory 7 for storing software and data from the sensor 5 and a processing unit 8 for executing software and for communicating with sensor 5 of the device. The processing unit 8 may have a clock for determining time points. The memory 7 is preferably a solid-state memory. The processing unit 8 may able to generate error codes if parts of the medical device malfunctions. Error codes may relate to, for example, that the battery does not charge, loss of communication with parts of the device, processing unit failure, memory failure or sensor failure.

The processing unit 8, memory 7 and cellular radio transceiver is powered by battery 20. The battery may also power sensor 5. The medical device 1 may also have a user interface that includes a display. The medical device 1 preferably has a housing that includes the various components of the medical device 1. The housing may have at least one opening to allow outside air to reach a sensor 5.

The sensor 5 may be a temperature sensor that is capable of measuring the temperature in the medical device 1 or in the immediate surroundings of the medical device 1. Any suitable type of temperature sensor may be used. The temperature sensor may be, for example, a thermocouple sensor or resistance temperature detector.

The sensor 5 may be a humidity sensor that is capable of measuring the humidity in the medical device 1 or in the immediate surroundings of the medical device 1. Any suitable kind of humidity sensor may be used. For example, a capacitive or a resistive hygrometer can be used.

The sensor 5 may be an accelerometer. The accelerometer may be a piezoelectric, piezoresistive or a capacitive accelerometer. The accelerometer may be used to determine if the medical device 1 has been exposed to a mechanical shock, for example if it has been dropped on the floor. The accelerometer may be a free fall sensor. A free fall sensor is activated when the medical device is in free fall and measures the time in free fall. The time parameter may be used to calculate the shock upon impact.

The sensor 5 may be an operations counter 14. The operations counter 14 may register the number of times the device has been used, i.e. the number of times that the medical device has injected a medicament. The operations counter 14 may detect the amount of medicament that is injected on each occasion. The operations counter 14 can for example be arranged to detect rotation of a rotating part of the injection means of the medical device. Examples of how a sensor 5 which is an operations counter 14 can be arranged can be found in WO2015076745 and references cited therein.

The operations counter 14 may comprise an magnetic or optical sensor that detects motion. The operations counter 14 may detect rotation or linear motion, for example linear motion of a plunger that expels medicament.

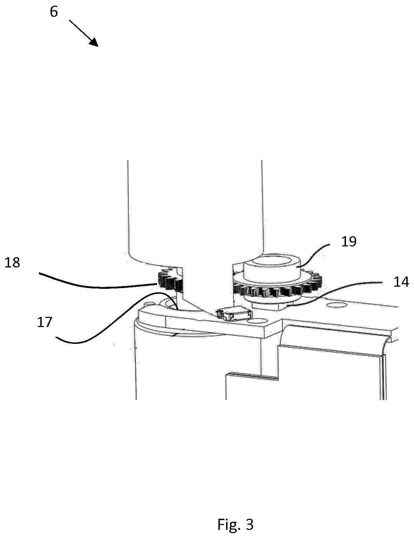

The operations counter 14 may be arranged as follows, when it is a sensor that detects the amount of medicament that has been injected by the device. Operations counter 14 is connected to the drive mechanism of the injection means 6 such that it can send a signal to the processing unit 8 that is proportional to the amount of medicament that is injected by the injection means of the device. An example of the arrangement of such a sensor is shown in Fig. 3, which shows an operations counter 14 that detects rotation of turning part 17 of the drive mechanism. Turning part 17 which is a part of the drive mechanism has a rim which forms a cogwheel 18 to which cogwheel 19 is connected. Turning of cogwheel 19 is detected by sensor 14 which may be a magnetic sensor. Operations counter 14 is able to send a signal to the processing unit 8. When the user presses the actuation means, the turning part 17 turns during injection of medicament. Turing of turning part 17 causes turning of cogwheels 18 and 19. The turning of the cogwheel 19 and the signal from the magnetic sensor 14 is proportional to the turning of turning part 17 and thus to the amount of medicament that has been injected.

The number of times the medical device 1 has been used may be detected by processing unit 8 by registering each time sensor 14 sends a signal regarding an operation to processing unit 8.

Measurements from the sensor 5 are stored in digital form in the memory 7 as sensor data. The processing unit 8 can retrieve the sensor data from the memory 7 and transfer it to the remote computer 4 with aid of cellular transceiver 2.

The sensor 5 is connected to processing unit 8 such that signalling can occur between these. Sensor measurement, sensor signal processing, and communication between the sensor 5 and the processing unit 8 and memory 7 can be carried out in any suitable manner known in the art. Signal processing and data storage may be carried out in various parts of device 1 such as in sensor 5, memory 7 and processing unit 8. When the output signal from the sensor 5 is an analogue signal, the processing unit 8 suitably has a capability for converting the signal to a digital signal, such and analogue-to-digital converter (ADC).

The sensor data generated by sensor 5 and/or processing unit 8 is stored in the memory 7 of the medical device 1. Each sensor 5 may have a separate memory 7 that is queried by the processing unit 8 at times.

Sensor 5 may be able to sense a parameter and send to the processing unit 8 data representing a numerical value of the parameter being measured. Thus the actual value of the temperature, humidity or acceleration or injection volume can be sent to processing unit 8 or memory 7 by sensor 5. Alternatively a value or signal that can be converted to obtain a temperature value, a humidity value, an acceleration value or an injection volume value is sent by sensor to the device 1 to the remote computer 4.

The data being transferred to the remote computer 4 from the medical device 1 is preferably in the form of numerical data (as opposed to categorical data). The sensor data may be a continuous variable (temperature, humidity, volume of medicament, acceleration, time in free fall) of the parameter being measured.

When the parameter is temperature, the sensor data is a value that is the temperature as measured in, for example, degrees Celsius or degrees Kelvin, or a value that can be converted, by the remote computer 4, to a temperature measurement.

When the parameter is humidity the sensor data is preferably a value that is the humidity as measured as a percentage of maximum relative humidity, or a value that can be converted to a percentage of maximum relative humidity measurement value by the remote computer 4.

When the parameter is number of operations the sensor data is preferably a value that represents the amount of injected medicament or the number of revolutions of a part of the injection means, or a value that can be converted to such a value by the remote computer 4.

When the parameter is acceleration the sensor data may be a value that represents acceleration as measured in m/s² or time in free fall, or a value that can be converted to such a value by the remote computer 4.

The medical device 1 and a remote computer 4 are parts of system 50. The medical device 1 typically operated and carried by a patient and the remote computer 4 is typically operated by a healthcare provider, a hospital or a company. The medical device 1 can establish a data connection with remote computer 4 and transfer sensor data to the remote computer 4. The remote computer 4 has a memory for storing software and data. The remote computer 4 further has a processor and is able to execute software, and it may have an operating system. The remote computer 4 may have a user interface 22 for allowing a user at the health care provider to change the settings of the remote computer, for example changing thresholds or receiving notifications. The remote computer 4 may be a server. Remote computer 4 can be two more computers or servers that cooperates.

The remote computer 4 can receive sensor data from the medical device 1 through the data connection that involves the cellular network 3. Preferably the remote computer 4 can retrieve data from a plurality of medical devices 1. Thus, the system 50 preferably includes a plurality of medical devices 1, which may be of the same model. The remote computer 4 can be used to monitor a plurality of medical devices 1. Each medical device 1 is associated with one patient/user.

The remote computer 4 has a communication interface 9 for communicating with the medical device 1 via the cellular network 3 or other computers. The data connection between the medical device 1 and the remote computer 4 can be used for transferring, from the medical device 1 to the remote computer 4, at least sensor data and the identity of the medical device. Optionally, the time point for the sensor measurement, or error codes generated by the medical device 1 can also be transferred by using the data connection.

The remote computer 4 has decision rule software 10 for making a decision about the medical device 1 while using sensor data from the medical device 1 as input. The decision may be if the medical device should be or serviced, scrapped or replaced, or that no action should be taken. The decision rule software 10 may comprise a threshold value for a type of sensor data. Thus there may be a threshold for each of temperature, humidity and acceleration or number of operations. The decision rule software 10 may be updated if needed. For example, a threshold value may be adjusted upwards or downwards by updating the decision rule software 10.

The remote computer 4 also comprises a database 11 that comprises the identities of a plurality of medical devices 1. Each medical device 1 has a unique identity that may be, for example, a number, a combination of letters or a combination of numbers and letters. The portable medical device 1 may have the identity stored in the memory 7 and is able to identify itself to the remote computer 4 by using the identity. The remote computer 4 can receive data from the medical device 1 and store it the database 11. Thus it is ensured that data for a particular medical device 1 is connected to that particular medical device 1 in the database 11.

Typically the medical device 1 is personal i.e. it is used by one person only. The database 11 preferably includes information that allows a health provider to get in touch with the user of the medical device 1. For example, the health provider may decide that a certain medical device 1 needs service or replacement, and then needs to inform the user regarding this.

The database 11 may contain information about the total use of a certain medical device 1 (total operations value). The total operations value is based on data from the operations counter 14 of the medical device 1. The total operations value may be represented by the total number of injections carried out or the total amount of injected medicament by a certain medical device 1. For this purpose the remote computer 4 may have operations tracker software 13. The operations tracker software 13 is able to receive data from the operations counter 14 and from the database 11, and to add the data from the operations counter 14 to the current total operations value stored in the database 11 to obtain an updated total operations value. For example, if the medical device on one occasion ejects 2 ml of medicament, and the device previously has injected a total of 200 ml (current total operations value) of medicament and the 200 ml of medicament has been injected at a total of 100 occasions (current total operations value), the operations tracker software 13 adds the data from the last event to the previously stored total so that the total amount of injected medicament will be 202 ml (updated total operations value) and the total number of injection events will be 101 (updated total operations value). The updated total operations values (202 ml and/or 101 occasions) is then stored in the database 11.

Alternatively, the operations tracker software 13 can be in the processing unit 8 of the medical device 1. The memory 7 of the medical device 1 then maintains a record of the total operations value. The total operations value can be updated as described above. The medical device 1 transfers the updated total operations value to the remote computer 4 at a suitable time point. The updated total operations value is then stored in the unit database 11 of remote computer 4 and used by decision rule software 10 at a suitable time point.

The threshold in the decision rule software 10 may be based on the number of injections or the total amount of medicament injected by the device 1.

The threshold or thresholds in the decision rule software 10 may be altered when it is necessary to do so. For example, if it is discovered, based on experience, that the threshold temperature should be lowered, this can be done in a convenient manner by the health provider by simply changing the threshold value in the decision rule software 10 of the remote computer 4.

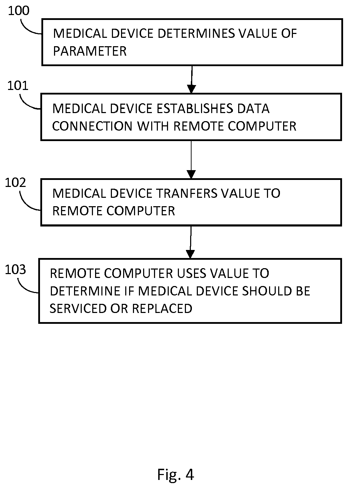

A method for monitoring the health of a medical device 1 will now be described with reference to Fig 4. In step 100 the sensor 5 of the medical device 1 establishes a value of the parameter being measured by the sensor 5. The sensor 5 may automatically measure temperature or humidity at predetermined intervals, for example once every 5 minutes, once per hour, once per day - or at the time of injection, or when the device is switched on by the user. The temperature or humidity at the time of injection may be more relevant since the medical device may be more sensitive to extreme conditions when it is in operation. Measurement may occur at the time of injection but at least once per every predetermined time interval, for example once per day.

The operations counter 14 detects operation at the time of injection of medicament.

When the sensor 5 is an accelerometer, acceleration may be continuously measured and data may be stored in memory 7 if the acceleration exceeds a certain threshold. Alternatively, the accelerometer is automatically activated if acceleration exceeds a certain threshold, such as beeing in free fall. The number of times a threshold is exceeded may be a value that is transferred to remote computer. This may allow remote computer 4 to count the number of steps the user has taken, for example.

In step 101 the medical device 1 uses the cellular radio transceiver 2 to connect to the cellular network 3 and establish a data connection with the remote computer 4. This step may involve switching on the cellular radio transceiver 2 or waking it up from sleep and carrying out a handshake with the cellular network 3, and then establishing a data connection with remote computer 4. Data communication between the remote computer 4 and the medical device 1 may be carried out using any suitable protocol or technology. Data to be transferred to the remote computer 4 is transferred to the cellular radio transceiver 2 from the processing unit 8 in a digital format.

Step 101 or step 102 comprises the step of the medical device 1 identifying itself to the remote computer 4 which may involve transferring the identity of the medical device 1 to the remote computer 4. This enables the remote computer 4 to store the received data for the correct individual medical device 1 in the unit database 11.

In step 102 the medical device 1 uses the data connection to transfer the sensor value determined in step 100 to the remote computer 4 so that the sensor data can be stored in the unit database 11 of the remote computer 4. The sensor data is stored in the unit database 11 and is logically connected to the identity of the medical device 1 in the unit database 11. The medical device 1 may also transfer information about the time point on which the sensor 5 determined the value and/or a time point of injection of medicament. Step 102 may involve the transfer of any error codes, and time points for these, generated by processing unit 8.

Steps 101 and 102 may be carried out as often as required, which may be once or a few times per day. Typically steps 101 and 102 are carried out immediately after injection has occurred. Steps 101 and 102 may be triggered by action of injection means 6. Injection mean 6 may send a signal regarding this to processing unit 8. The signal may be a signal from operations counter 14. Thus operations counter 14 may trigger steps 101 and 102. Typically step 102 is carried out immediately after step 101, typically as soon as possible after step 101 in order to save battery power. In order to save battery, steps 101 and 102 does not have to be carried out every time new sensor data has been determined.

Data from an operations counter 14 may in addition be used at remote computer 4 to monitor the behaviour of patients, for example to monitor patient compliance, and it is convenient to transfer any other sensor data to the remote computer 4 at the same time, in the same communications session, in order to save battery.

When data has been transferred, the cellular radio transceiver 2 may be immediately switched off or put in sleep mode in order to save battery power.

In step 103 the remote computer 4 uses the sensor value from step 102 to decide whether the portable medical device should be serviced or replaced, or if no action should be taken. Optionally, this may involve the operations tracker software 13 calculating a total use as described above. In a basic embodiment, the decision rule software 10 compares the sensor data from a sensor 5 (or from the operations tracker software 13) with a predetermined threshold value.

Step 103 may suitably be carried out each time the remote computer 4 receives data, preferably immediately after step 102.

When the parameters being measured is temperature the threshold value may be a minimum or a maximum temperature.

When the parameter being measured is humidity, the threshold value may be a maximum humidity.

When the parameter being measured is acceleration the threshold value may be a maximum acceleration, or maximum time in free fall. The maximum time in free fall determines a maximum impact shock to the medical device 1.

When the sensor 5 is an operations counter 14 the threshold value may be a threshold total operations value, for example the maximum number of injections. The threshold total operations value may alternatively be a maximum amount of injected medicament, for example total volume of injected medicament. Such a threshold may be set to indicate when the medical device has been worn out or otherwise used to the extent that it should be replaced or serviced.

If the value from the sensor 5 or the operations tracker software 13 exceeds the threshold (or - in the case of a minimum temperature - is lower than the minimum temperature), a decision is taken by the decision rule software 10 to classify the individual medical device 1 as in need of service or replacement. This may trigger further action, such as for example notifying the user or a health provider. It may also trigger the automatic shipment of a replacement medical device 1 to the user. The identity of the medical device is preferably tied to a particular user in the database 11.

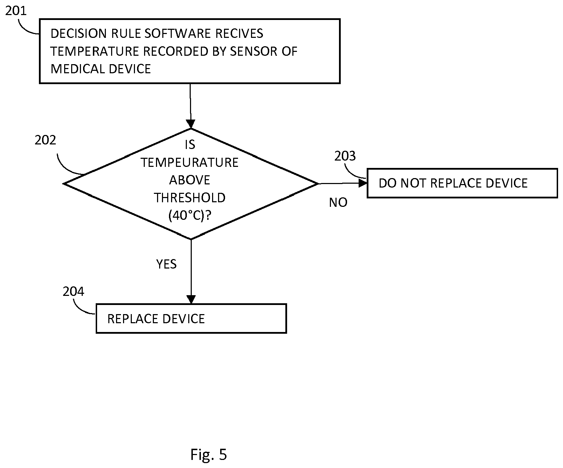

An example of this is shown in Fig. 5. In step 201 the decision rule software 10 of the remote computer 4 receives sensor data representing a temperature recorded by the sensor 5. In step 202 the decision rule software 10 applies the decision rule. In this case the decision rule is that if a temperature of above 40° has been recorded by the sensor 5, the medical device 1 should be replaced. If the recorded temperature value is found, in step 202, to be below or equal to 40° a decision is taken not to replace the device in step 203. If the temperature value from sensor 5 is above 40° a decision is taken in step 204 to replace the medical device 1. Thus if the medical device has been subjected to a temperature of above 40° a decision is taken by decision rule software 10 in step 204 to replace the device.

The output from the decision rule software 10 can be communicated from remote computer 4 by using the communications interface 9 and/or user interface 22. The user interface 22 may provide a notification regarding replacement, scrapping or service of the medical device 1. Such a notification can be in any suitable form. The notification may be sent to a computer system that keeps tracks of a plurality of medical devices 1. Such a computer system may take the relevant action, for example, automatically ordering a new device, notifying or warning the user of the medical device, or notify or warn medical or technical staff. The remote computer may for example send a message to the user of the medical device 1, for example an email or SMS/text message to the mobile phone of the user.

The remote computer 4 may use a data connection to medical device 1 to send a signal to the medical device 1, the signal causing the medical device 1 to notify the user of the medical device that the medical device 1 needs service or replacement. The signal from the remote computer may cause the medical device 1 to carry out a systems check, for example check battery power. The signalling may preferably occur in the same communication session between the medical device 1 and remote computer 4. Alternatively, this occurs in a later communication session. The notification to the user may be a message displayed on a display of the medical device 1, or a sound signal, or vibration, or a combination of these, or by shutting down the medical device 1. The message on the display may be for example "Error" or "Service" or "Contact health provider X", or "Call XX-XXXXX". The medical device may thus have a user interface, which may include a display, which can be used for the notification.

The method may involve the additional step of determining the geographical position of the medical device 1. The geographical position can be used for retrieving data that is not detected by a sensor of the medical device 1. For example, if the medical device 1 does not have a humidity sensor, the geographical position, together with a time point, can be used by remote computer 4 for retrieving humidity data from a weather service. This is described in more detail below.

The geographical position of the medical device 1 can be determined in various manners, for example with a GPS circuit in the medical device 1 or by using the cellular radio transceiver 2 for mobile phone tracking. Mobile phone tracking is preferred since the medical device 1 has a cell radio transceiver 2. The accuracy of mobile phone tracking is sufficient for most purposes. When mobile phone tracking is used, the position of the mobile device is determined by the cellular network 3. The cellular network 3 may use information about which cell the medical device 1 is connected to, or may use multilateration involving several cells. The position data is sent from the cell network 3 to the remote computer 4 in any suitable manner. For example the remote computer 4 may query a computer of the cell network 3 for geographical data regarding a particular medical device 1. The remote computer 4 can store the position data in the database 11. The time point for determining the geographical position is preferably immediately when the medical device 1 connects to the cellular network 3, or within a predetermined time limit of a sensor measurement. The time limit can, for example, be a time of up to 30 minutes.

Typically the medical device 1 will connect to the cellular network 3 immediately after injection has been carried out. The approximate geographical position of where injection was carried out will then be determined and transferred to the remote computer 4.

Alternatively, position data generated by a mobile phone that is carried by the user can be used, using for example GPS and/or Wi-Fi positioning ability of the phone. It is then assumed that the user carries the mobile phone and the medical device 1 with himself or herself. Such position data can be sent to the remote computer 4 from the mobile phone and paired with a sensor measurement, for example by operations counter 14 by comparing time points. Thus the remote computer 4 can select the geographical position as determined by the mobile phone that is closest in time to the time point of the sensor measurement. For example, if a sensor measurement takes place at 15.25 and there is a position X at 15.00 and a position Y at 1530, the remote computer 4 selects position Y, since it is closer in time to the sensor measurement.

In one embodiment the mobile phone has software (for example an app) that automatically determines the position and stores this in the memory of the mobile phone. This can be repeated on at least suitable predetermined time intervals, for example at least every 15 minutes. The position data is transferred to the remote computer 4 on at least suitable (longer) predetermined time intervals points, for example once per day.

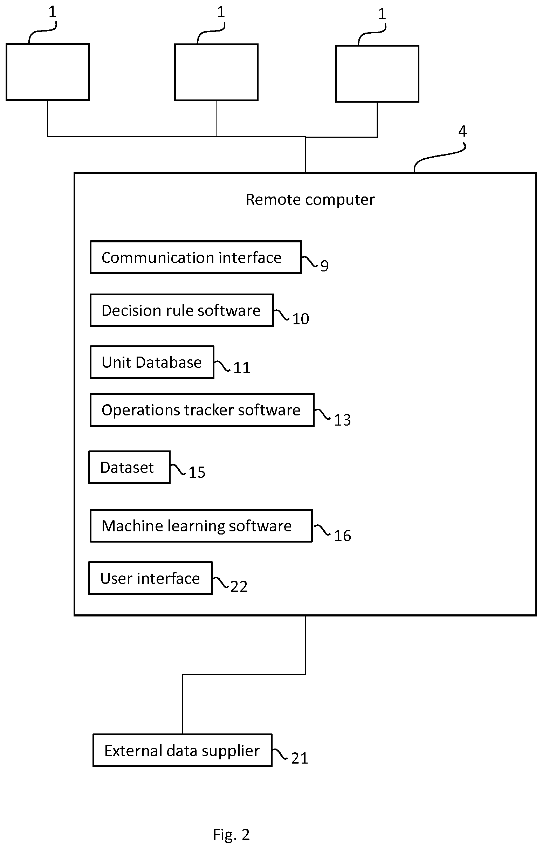

With reference to Fig. 2, in a preferred embodiment the decision rule software 10 implements a decision rule that is generated by applying machine learning to a data set 15, said data set 15 comprising data previously collected from similar portable medical devices, said data set 15 comprising data about any previous failure of the similar devices 1. Failures includes breakdowns, failure, replacements or error codes of such similar portable medical devices. "Similar" or "comparable" devices means devises are devices that can be compared for example because they are of the same model or shares certain component in a way that one can reasonable expect that they behave in the same manner.

Machine learning, as such, is previously known. Machine learning may for example involve applying Bayesian networks or artificial neural networks. Guidance can be found in US2014/0012784, US2008/0059120 and US8819498. The remote computer 4 may comprise machine learning software 16 that analyses the data set 15 to produce a decision rule that is implemented in the decision rule software 10. The data set 15 may comprise the information in the unit database 11.

The dataset 15 may comprise any useful information. Thus the data set 15 may include sensor data, information about model, serial number, address of user, manufacturing day, components and manufacturer of the medical device 1.

Importantly, the dataset 15 comprises information about previous failures of medical devices 1 or similar medical devices. This data may be entered manually. Alternatively the dataset 15 may automatically receive error codes from the medical devices 1 or comparable medical devices. The size of the dataset 15 will increase overtime, which will improve the decision rule. The method may involve the step of automatically adding the sensor data to the dataset 15. The method may involve the step of automatically adding information about error codes from the medical device 1 to the dataset 15.

By applying machine learning to the dataset 15, the decision rule of the decision rule software 10 will be able to take into account previously unknown factors for diagnosing the medical device 1. For example, it is feasible that a certain pattern of use (say high frequency use) together with a low temperature may increase the risk of damage to the medical device. It is possible to detect such a failure pattern by using machine learning. Thus the method can be used as a research tool. Parameters that can be included in the dataset 15 and thus be taken into account when the machine learning software 16 creates decision rule in the decision rule software 10 may include: temperature, humidity, acceleration, usage pattern (frequency and amount, for example), error codes, time of day for use or time of day for generation of error code, model, serial number, address of user, manufacturing day, serial number of components in the medical device 1, and replacement of the medical device 1.

By applying machine learning to the dataset new patterns that predict failure of the medical device 1 can be discovered. Such patterns are yet to be discovered but a few purely hypothetical examples are now mentioned for illustration. A first hypothetical example is that injection at high temperatures predicts wear of the medical device 1. A second hypothetical example is that a combination of high temperature and high humidity results in failure of the medical device when the medical device has parts from a certain subcontractor. A third example is that injection of volumes over 1 ml causes battery charging error codes.

Preferably the dataset 15 includes data from a large number of medical devices 1, preferably more than a thousand medical devices 1.

The method may comprise the step of the remote computer 4 adding the information, in particular sensor data, received from the medical device 1 to the dataset 15.

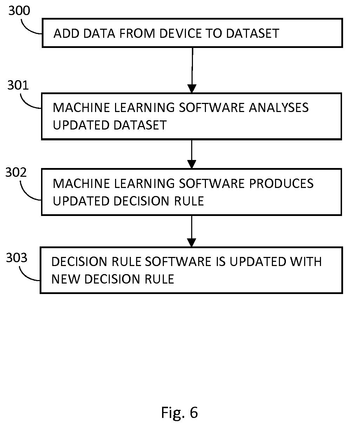

The method may comprise the step of the machine learning software 16 analysing the updated dataset 15 and producing an updated decision rule, and updating the decision rule software 10 with the new decision rule. This is shown in Fig. 6 which shows how in step 300, the remote computer 4 receives data, for example values data, from a device 1. The data is then added to the data set 15. In step 301 machine learning software 16 analyses the updated dataset 15, to produce an updated decision rule in step 302. This updated decision rule is then used to update the decision rule software 10 in step 303. The machine learning software 16 may apply a cutoff for providing a decision rule such that a decision rule must have at least a minimum predetermined confidence in order for the decision rule to be provided to be used for updating the decision rule software. Step 303 may be omitted. When step 303 is omitted the decision rule software may be updated manually. The machine learning software 16 may then send a notification, using user interface 22, to an operator who may then update decision rule software automatically.

The geographical position at a certain time point of the medical device 1, possibly together with a time point for injection or sensor measurement, may be used to gather information that is useful for including in the dataset 15. Such information may include for example weather, temperature, humidity, air pollution, air pressure, calendar data, or road traffic information. An external provider of data 21 may provide such information to the remote computer 4. For example, weather information can be supplied by an external source of weather information. This is useful if the medical device 1 does not have a temperature or humidity sensor. Traffic information may provided by Google.

The external provider of data 21 may provide information about the user from a social network service, and such data may be included in the dataset 15. Social network services may extract such information based on the posts that user make. Extraction of such data from a social network service typically requires that the user allows such extraction. Types of behaviour may include sport activities, social activities and travel. A time point, for example a time point close to the time of injection, is sufficient for extracting this type of data. A social network at least allows a second user to manually input information using a client device, transferring the information to a social network service server and making the information available to a plurality of other users. Each user has an account enabling connection to the server. Each account has a unique user name and a password.

The measurement by the temperature sensor or humidity sensor may be activated by the user activating the injection actuator, or activating the dose setting means or by the user switching on the medical device 1. Thus, the device may comprise an activation switch 23 whereby a user can cause the humidity or temperature sensor 5, to measure temperature or humidity and store the temperature value or humidity value in the memory 7 of the medical device 1.

Sensoring and transfer of data may be carried out in a session where the device 1 is switched on, injection occurs, measurement of temperature or humidity and transfer of data to the remote computer 4 takes place. Measurement of temperature and humidity may be triggered by switching on the device 1 and transfer of sensor data (including data from operations counter 14) to remote computer 4 may be triggered by an injection, or other user action.

The activation switch 23 may for example be an on/off button which the user can use to switch on the device 1 or wake up the device 1 from sleep. The activation switch 23 may also be switched to "on" by use of the medical device 1. For example, the activation switch 23 may be the dose setting means, such that when the user sets the dose to be injected the device 1 is switched on or is waken up from sleep. For example, when the user sets the dose to another value than zero, the device 1 may be switched on or waken up from sleep, and cause the processing unit to obtain a measurement from the humidity or temperature sensor. Other types of activation switches 23 are also possible. The device may have one, two, three or more activation switches that all are capable of switching on the device 1.

The device 1 can be configured to, when the user has used the activation switch 23, to wait for further user action during a predetermined waiting period, and, if the user takes further action within the waiting period, establish a data connection to the remote computer 4 and transfer the determined value to the remote computer 4, and if the user does not take further action within the waiting period, being configured to transfer the determined value the next time a data connection is established with the remote computer 4.

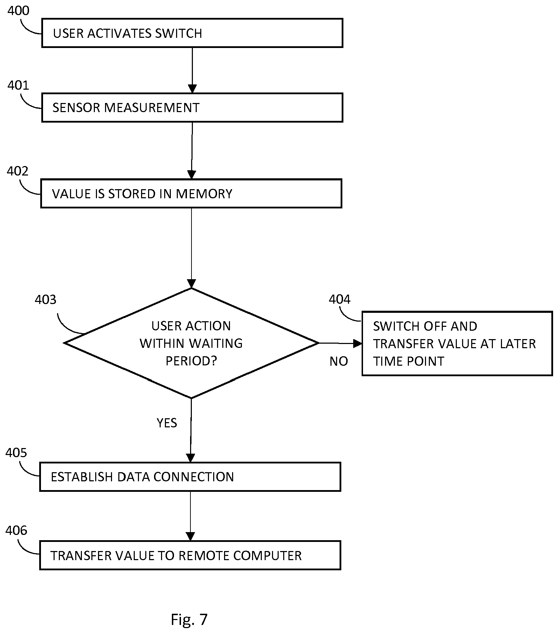

This is described in Figure 7. In step 400 the user activates the activation switch 23, for example by setting a dose setting means to another value than zero or by switching on the device 1 with an on/off button. The cellular radio transceiver 2 may be switched on at this time point, but set to listening mode only (this enables determining of position) in order to save battery power. Thus cellular radio transceiver does preferably not carry out a handshake with cellular network 3 at this point. In step 401, a temperature sensor 5 or humidity sensor 5 carries out a measurement and the value from this measurement is, in step 402, stored in the memory 7 of device 1. The value is preferably stored together with a time point for measurement. Steps 401 and 402 are preferably carried out immediately after step 400. Either of steps 400, 401 or 402 can trigger the waiting period of step 403. The waiting period is preferably predetermined and may be, for example, from 10 seconds to 5 minutes. A suitable waiting period may be 30 seconds. The waiting period is measured by a timer in device 1, for example a timer in processing unit 8. If the user does not take any action within the waiting period, the device may be automatically switched off in or go to sleep mode in step 404. The stored sensor value may then be transferred the next time a computer connection is established with remote computer 4.

If the user takes further action within the waiting period, a data connection is established with remote computer in step 405 and the value from sensor 5 is then transferred to the remote computer in step 406. The further action that triggers establishing the data connection may be for example making an injection. This may be sensed by an operations sensor 14. The further action may also be the user manually making input to the processing unit 8 of the medical device. The medical device may have an electronic or digital log for logging time points for meals, exercise and/or illness and making input to such a digital log may trigger data transfer to remote computer. Such an electronic or digital log is described in WO2015076745. Data regarding further actions is also transferred to the remote computer 4.

Thus, in this embodiment, data from temperature sensor 5 or humidity sensor 5 is only transferred together with data created when the user takes further action. Carrying out a handshake with cellular network 3 is energy consuming and the purpose of the waiting period is to save battery power of the medical device 1. Thus, in this embodiment, value from the temperature sensor or humidity sensor is transferred to remote computer 4 only when the user takes further important action such as making an injection or inputting data into the user log. The later kind of actions results in data that needs to be speedily transferred to remote computer 4 since remote computer 4 may also be used for monitoring patient health and compliance.

In the memory 7 of device, sensor data may be tagged as transferred or non-transferred in order for the device to keep track of which sensor data has been transferred to the remote computer 4. Or alternatively, in a data transfer session, the device 1 transfers all sensor data that has not been transferred since the time point for last sensor data transfer.

The methods and devices herein can be implemented with any suitable combination of hardware and software.

While the invention has been described with reference to specific exemplary embodiments, the description is in general only intended to illustrate the inventive concept and should not be taken as limiting the scope of the invention. The invention is generally defined by the claims.

## Claims

1. A method involving a portable medical device (1) for the injection of a medicament, said medical device (1) comprising means (6) for injection of a medicament, said medical device (1) further comprising a cellular radio transceiver (2) and a sensor (5) that is an operations counter (14), said portable medical device further comprising an injection actuator which a user can use to self-administer a medicament, the method comprising the steps of
a) the portable medical device (1) using the sensor (5) to determine a value for a parameter that is sensed by the sensor (5), the parameter being the operations counter detecting an injection, and a time point for injection,
b) the portable medical device (1) using the cellular radio transceiver (2) to establish a data connection to a remote computer (4) with the use of a cellular network (3),
c) the portable medical device (1) using the data connection to transfer the data from step a) to the remote computer (4),
d) determining the geographical position of the portable medical device (1), and transferring information about the geographical position to the remote computer (4),
e) the remote computer using the geographical position and the time point to retrieve data for at least one additional parameter,
f) the remote computer (4) using the data from step e) to determine whether the portable medical device 1 is in need of scrapping, service or replacement,

2. The method according to claim 1 where the parameter is a parameter selected from the group consisting of temperature, humidity, weather, calendar data, air pollution, air pressure and road traffic information.

3. The method according to any one of claim 1 to 2 where step f) involves the remote computer (4) establishing if the value exceeds a threshold value.

4. The method according to any of claims claim 1 to 3 where step f) involves the remote computer (4) applying a decision rule, said decision rule being generated by applying machine learning to a data set (15), said data set (15) comprising data previously collected for comparable portable medical devices, said data set (15) also comprising data about previous failures of the comparable portable medical devices.

5. The method of claim 4 where the data set (15) comprises data for at least one parameter selected from the group consisting of temperature, humidity, acceleration or time in free fall, weather, calendar data, air pollution, air pressure, geographical position, number of operations, amount of medicament injected, time of injection, geographical position at time of injection.

6. The method according to any one of claims 4 to 5 where the data in the dataset (15) has been added to the dataset (15) according to the method of any one of claims 1 to 11.

7. The method according to any one of claims 4 to 6 where the dataset (15) comprises information made available by users of comparative medical devices, said information being from a social network service, and the remote computer in step d) uses information regarding the user of the portable device (1) for which the decision rule is applied, said information made available by the user to the social network service.

8. The method according to any one of claims 1 to 7 where the injection actuator comprises a button that can be pressed by a user.

9. A system (50) for deciding if a portable medical device (1) for the injection of a medicament should be serviced or replaced, said system (50) comprising a portable medical device (1) for injection of a medicament and a remote computer (4), said portable medical device (1) comprising injection means (6), a processing unit (8), a memory (7) and a cellular radio transceiver (2) and at least one sensor (5) the sensor being an operations counter (14), said portable device further comprising an injection actuator which a user can use to self-administer a medicament, said portable medical device (1) being configured to use the operations counter to sense injection and to store a time point for injection, in the memory (7), said remote computer (4) and portable medical device (1) adapted to establish a data connection with the use of a cellular network (3), the portable medical device (1) being configured to use the data connection to transfer the time of injection to the remote computer (4), the system (50) being configured to determine the geographical position of the medical device (1) at the time point for injection, the remote computer (4) being configured to use the geographical position and the time point to retrieve data for a parameter and to use the retrieved data to decide whether the portable medical device is in need of scrapping, service or replacement

10. The system according to claim 9 where the remote computer (4) comprises decision rule software (10), said decision rule software (10) being generated by applying machine learning to a data set (15), said data set (15) comprising sensor values previously collected from comparable medical devices, said data set (15) further comprising data about any previous failures of the comparable portable medical devices.

11. The system according to any of claims 9 or 10 where the injection actuator comprises a button that can be pressed by a user.

12. A remote computer (4) being configured to receive, using a data connection, from a portable medical device (1) for the injection of a medicament, a value for a parameter, the parameter being a time point for injection, and receiving the geographical position of the medical device, the remote computer further being configured to use the geographical position and the time point to retrieve data for at least one additional parameter, and using the data to determine whether the portable medical device (1) is in need of scrapping, service or replacement.

13. The remote computer (4) according to claim 12 where the determination involves the remote computer (4) applying a decision rule, said decision rule being generated by applying machine learning to a data set (15), said data set (15) comprising sensor data previously collected from comparable portable medical devices, said data set (15) also comprising data about any previous failures of the comparable portable medical devices.

## Patentansprüche

1. Verfahren mit einem tragbaren medizinischen Gerät (1) zur Injektion eines Medikaments, wobei das medizinische Gerät (1) Mittel (6) zur Injektion eines Medikaments umfasst, wobei das medizinische Gerät (1) ferner einen Mobilfunktransceiver (2) und einen Sensor (5) umfasst, welcher ein Betriebszähler (14) ist, wobei das tragbare medizinische Gerät ferner einen Injektionsaktuator umfasst, welchen ein Benutzer verwenden kann, um sich selbst ein Medikament zu verabreichen, wobei das Verfahren die Schritte umfasst:
a) die tragbare medizinische Vorrichtung (1) verwendet den Sensor (5), um einen Wert für einen Parameter zu bestimmen, der von dem Sensor (5) erfasst wird, wobei der Parameter der Betriebszähler ist, welcher eine Injektion und einen Zeitpunkt für die Injektion erfasst,
b) das tragbare medizinische Gerät (1) verwendet den Mobilfunktransceiver (2), um eine Datenverbindung zu einem Computer mit Fernzugriff (4) durch die Verwendung eines Mobilfunknetz (3) herzustellen,
c) das tragbare medizinische Gerät (1) verwendet die Datenverbindung zum Übertragen der Daten aus Schritt a) zu dem Computer mit Fernzugriff (4),
d) Bestimmen der geografischen Position des tragbaren medizinischen Geräts (1) und Übertragen von Informationen über die geografische Position an den Computer mit Fernzugriff (4),
e) der Computer mit Fernzugriff verwendet die geografische Position und den Zeitpunkt, um Daten zumindest eines zusätzlichen Parameters abzurufen,
f) der Computer mit Fernzugriff (4) verwendet die Daten aus Schritt e), um zu bestimmen, ob das tragbare medizinische Gerät (1) verschrottet, gewartet oder ersetzt werden muss.

2. Verfahren gemäß Anspruch 1, wobei der Parameter ein Parameter ausgewählt aus der Gruppe bestehend aus Temperatur, Feuchtigkeit, Wetter, Kalenderdaten, Luftverschmutzung, Luftdruck und Straßenverkehrsinformation ist.

3. Verfahren gemäß einem der Ansprüche 1 bis 2, wobei Schritt f) beinhaltet, dass der Computer mit Fernzugriff (4) festschreibt, ob der Wert einen Grenzwert überschreitet.

4. Verfahren gemäß einem der Ansprüche 1 bis 3, wobei Schritt f) beinhaltet, dass der Computer mit Fernzugriff (4) eine Entscheidungsregel anwendet, wobei die Entscheidungsregel durch Anwendung von maschinellem Lernen auf einen Datensatz (15) erzeugt wird, wobei der Datensatz (15) Daten von zuvor erfassten vergleichbaren tragbaren medizinischen Geräten umfasst, wobei der Datensatz (15) auch Daten über frühere Ausfälle vergleichbarer tragbarer medizinischer Geräte umfasst.

5. Verfahren gemäß Anspruch 4, wobei der Datensatz (15) Daten umfasst, für mindestens einen Parameter ausgewählt aus der Gruppe bestehend aus: Temperatur, Feuchtigkeit, Beschleunigung oder Zeit im freien Fall, Wetter, Kalenderdaten, Luftverschmutzung, Luftdruck, geographische Position, Anzahl der Operationen, Menge des injizierten Medikaments, Zeitpunkt der Injektion, geographische Position zum Zeitpunkt der Injektion.

6. Verfahren gemäß einem der Ansprüche 4 bis 5, wobei die Daten in dem Datensatz (15) zu dem Datensatz (15) gemäß dem Verfahren nach einem der Ansprüche 1 bis 11 hinzugefügt wurden.

7. Verfahren gemäß einem der Ansprüche 4 bis 6, wobei der Datensatz (15) Informationen umfasst, die von Benutzern vergleichbarer medizinischer Geräte zur Verfügung gestellt wurden, wobei die Informationen von einem sozialen Netzwerkdienst stammen und der Computer mit Fernzugriff in Schritt d) Informationen verwendet, betreffend den Benutzer des tragbaren Geräts (1), für das die Entscheidungsregel angewendet wird, wobei die Informationen durch den Benutzer dem sozialen Netzwerkdienst zur Verfügung gestellt wurde.

8. Verfahren gemäß einem der Ansprüche 1 bis 7, wobei der Injektionsakuator einen Knopf umfasst, der von einem Benutzer gedrückt werden kann.

9. System (50) zur Entscheidung, ob ein tragbares medizinisches Gerät (1) zur Injektion eines Medikaments gewartet oder ersetzt werden soll, wobei das System (50) eine tragbare medizinische Vorrichtung (1) zur Injektion eines Medikaments und einen Computer mit Fernzugriff (4) umfasst, wobei das tragbare medizinische Gerät (1) umfasst: ein Mittel zur Injektion (6), eine Verarbeitungseinheit (8), einen Speicher (7) und einen Mobilfunktransceiver (2) und zumindest einen Sensor (5), wobei der Sensor ein Betriebszähler (14) ist, wobei das tragbare Gerät ferner einen Injektionsaktuator umfasst, welchen ein Benutzer verwenden kann, um sich selbst ein Medikament zu verabreichen, wobei das tragbare medizinische Gerät (1) so konfiguriert ist, dass es den Betriebszähler nutzt um eine Injektion zu erfassen und einen Zeitpunkt der Injektion in den Speicher (7) zu speichern, wobei der Computer mit Fernzugriff (4) und das tragbare medizinische Gerät (1) dazu ausgelegt sind, eine Datenverbindung mit der Verwendung des Mobilfunknetzes (3), herzustellen, wobei das tragbare medizinische Gerät (1) dazu konfiguriert ist, die Datenverbindung zu verwenden, um die Injektionszeit an den Computer mit Fernzugriff (4) zu übertragen, wobei das System (50) dazu konfiguriert ist, die geografische Position des medizinischen Geräts (1) zum Zeitpunkt der Injektion zu bestimmen, wobei der Computer mit Fernzugriff (4) dazu konfiguriert ist, die geografische Position und den Zeitpunkt zu verwenden, um Daten für einen Parameter abzurufen und die abgerufenen Daten dazu zu verwenden, um zu entscheiden, ob das tragbare medizinische Gerät (1) verschrottet, gewartet oder ersetzt werden muss.

10. System nach Anspruch 9, wobei der Computer mit Fernzugriff (4) eine Entscheidungsregelsoftware (10) umfasst, wobei die Entscheidungsregelsoftware (10) durch eine Anwendung eines maschinellen Lernens auf einen Datensatz (15) generiert wird, wobei der Datensatz (15) Sensorwerte umfasst, welche zuvor von vergleichbaren medizinischen Geräten erfasst wurden, wobei der Datensatz (15) zusätzlich Daten über etwaige frühere Ausfälle vergleichbarer tragbarer medizinischer Geräte umfasst.

11. System nach einem der Ansprüche 9 oder 10, umfassend einen Knopf, der von einem Benutzer gedrückt werden kann.

12. Computer mit Fernzugriff (4), der dazu konfiguriert ist, unter Verwendung einer Datenverbindung, von einem tragbaren medizinischen Gerät (1) für die Injektion eines Medikaments einen Wert für einen Parameter zu empfangen, wobei der Parameter ein Zeitpunkt für die Injektion ist, und die geografische Position des medizinischen Geräts zu empfangen, wobei der Computer mit Fernzugriff zusätzlich dazu konfiguriert ist, die geografische Position und den Zeitpunkt dazu zu verwenden, um Daten für mindestens einen zusätzlichen Parameter zu ermitteln, und die Daten dazu zu verwenden, um zu bestimmen, ob das tragbare medizinische Gerät (1) verschrottet, gewartet oder ersetzt werden muss.

13. Computer mit Fernzugriff (4) gemäß Anspruch 12, wobei die Bestimmung den Ferncomputer (4) beinhaltet, der eine Entscheidungsregel anwendet, wobei die Entscheidungsregel durch die Anwendung von maschinellem Lernen auf einen Datensatz (15) erzeugt wird, wobei der Datensatz (15) Sensordaten umfasst, die zuvor von vergleichbaren tragbaren medizinischen Geräten gesammelt wurden, wobei der Datensatz (15) auch Daten über etwaige frühere Ausfälle der vergleichbaren tragbaren medizinischen Geräte umfasst.

## Revendications

1. Procédé concernant un dispositif médical portable (1) permettant l'injection d'un médicament, ledit dispositif médical (1) comprenant des moyens (6) destinés à l'injection d'un médicament, ledit dispositif médical (1) comprenant en outre un émetteur-récepteur radiocellulaire (2) et un capteur (5) qui est un compteur d'opérations (14) ledit dispositif médical portable comprenant en outre un actionneur d'injection qu'un utilisateur peut utiliser pour auto-administrer un médicament,
le procédé comprenant les étapes consistant à :
a) au moyen du dispositif médical portable (1), utiliser le capteur (5) pour déterminer une valeur de paramètre qui est détecté par le capteur (5), le paramètre étant la détection d'une injection par le compteur d'opérations et un instant de l'injection,
b) au moyen du dispositif médical portable (1), utiliser l'émetteur-récepteur radiocellulaire (2) pour établir une connexion de données à un ordinateur distant (4) à l'aide d'un réseau cellulaire (3),
c) utiliser la connexion de données au moyen du dispositif médical portable (1) pour transférer les données issues de l'étape a) vers l'ordinateur distant (4),
d) déterminer la position géographique du dispositif médical portable (1), et transférer des informations au sujet de la position géographique vers l'ordinateur distant (4),
e) au moyen de l'ordinateur distant, utiliser la position géographique et l'instant pour récupérer des données relatives à au moins un paramètre supplémentaire,
f) au moyen de l'ordinateur distant (4), utiliser les données issues de l'étape e) pour déterminer si le dispositif médical portable (1) nécessite une mise au rebut, un entretien ou un remplacement.

2. Procédé selon la revendication 1, dans lequel le paramètre est un paramètre choisi dans le groupe composé de la température, de l'humidité, des conditions météorologiques, des données de calendrier, de la pollution atmosphérique, de la pression de l'air et des informations de circulation routière.

3. Procédé selon l'une quelconque des revendications 1 et 2, dans lequel l'étape f) implique d'évaluer au moyen de l'ordinateur distant (4) si la valeur dépasse une valeur de seuil.

4. Procédé selon l'une quelconque des revendications 1 à 3, dans lequel l'étape f) implique d'appliquer une règle de décision au moyen de l'ordinateur distant (4), ladite règle de décision étant engendrée par application d'un apprentissage machine à un ensemble de données (15), ledit ensemble de données (15) comprenant des données antérieurement collectées pour des dispositifs médicaux portables comparables, ledit ensemble de données (15) comprenant également des données au sujet de défaillances antérieures des dispositifs médicaux portables comparables.

5. Procédé selon la revendication 4, dans lequel l'ensemble de données (15) comprend des données relatives à au moins un paramètre choisi dans le groupe composé de la température, de l'humidité, de l'accélération ou du temps en chute libre, des conditions météorologiques, des données de calendrier, de la pollution atmosphérique, de la pression de l'air, de la position géographique, du nombre d'opérations, de la quantité de médicament injectée, du moment de l'injection, de la position géographique au moment de l'injection.

6. Procédé selon l'une quelconque des revendications 4 et 5, dans lequel les données de l'ensemble de données (15) ont été ajoutées à l'ensemble de données (15) conformément au procédé selon l'une quelconque des revendications 1 à 11.

7. Procédé selon l'une quelconque des revendications 4 à 6, dans lequel l'ensemble de données (15) comprend des informations mises à disposition par des utilisateurs de dispositifs médicaux comparatifs, lesdites informations émanant d'un service de réseau social, et l'ordinateur distant à l'étape d) utilise des informations concernant l'utilisateur du dispositif portable (1) pour lequel la règle de décision est appliquée, lesdites informations étant mises à disposition du service de réseau social par l'utilisateur.

8. Procédé selon l'une quelconque des revendications 1 à 7, dans lequel l'actionneur d'injection comprend un bouton pouvant être actionné par un utilisateur.

9. Système (50) destiné à décider si un dispositif médical portable (1) permettant l'injection d'un médicament doit faire l'objet d'un entretien ou d'un remplacement, ledit système (50) comprenant un dispositif médical portable (1) permettant l'injection d'un médicament et un ordinateur distant (4), ledit dispositif médical portable (1) comprenant des moyens d'injection (6), une unité de traitement (8), une mémoire (7) et un émetteur-récepteur radiocellulaire (2) ainsi qu'au moins un capteur (5) le capteur étant un compteur d'opérations (14), ledit dispositif portable comprenant en outre un actionneur d'injection qu'un utilisateur peut utiliser pour s'auto-administrer un médicament, ledit dispositif médical portable (1) étant conçu pour utiliser le compteur d'opérations afin de détecter une injection et pour stocker un instant de l'injection, dans la mémoire (7), ledit ordinateur distant (4) et ledit dispositif médical portable (1) étant destinés à établir une connexion de données à l'aide d'un réseau cellulaire (3), le dispositif médical portable (1) étant conçu pour utiliser la connexion de données afin de transférer le moment de l'injection vers l'ordinateur distant (4), le système (50) étant conçu pour déterminer la position géographique du dispositif médical (1) à l'instant de l'injection, l'ordinateur distant (4) étant conçu pour utiliser la position géographique et l'instant afin de récupérer des données relatives à un paramètre et pour utiliser les données récupérées afin de décider si le dispositif médical portable nécessite une mise au rebut, un entretien ou un remplacement.

10. Système selon la revendication 9, dans lequel l'ordinateur distant (4) comprend un logiciel de règle de décision (10), ledit logiciel de règle de décision (10) étant engendré par application d'un apprentissage machine à un ensemble de données (15), ledit ensemble de données (15) comprenant des valeurs de capteurs antérieurement collectées auprès de dispositifs médicaux comparables, ledit ensemble de données (15) comprenant en outre des données au sujet de défaillances antérieures quelconques des dispositifs médicaux portables comparables.

11. Procédé selon l'une quelconque des revendications 9 ou 10, dans lequel l'actionneur d'injection comprend un bouton pouvant être actionné par un utilisateur.

12. Ordinateur distant (4) conçu pour recevoir, au moyen d'une connexion de données, en provenance d'un dispositif médical portable (1) permettant l'injection d'un médicament, une valeur de paramètre, le paramètre étant un instant d'injection, et recevoir la position géographique du dispositif médical, l'ordinateur distant étant conçu en outre pour utiliser la position géographique et l'instant afin de récupérer des données relatives à au moins un paramètre supplémentaire, et utiliser les données pour déterminer si le dispositif médical portable (1) nécessite une mise au rebut, un entretien ou un remplacement.

13. Ordinateur distant (4) selon la revendication 12, dans lequel la détermination implique d'appliquer une règle de décision au moyen de l'ordinateur distant (4), ladite règle de décision étant engendrée par application d'un apprentissage machine à un ensemble de données (15), ledit ensemble de données (15) comprenant des données de capteurs antérieurement collectées auprès de dispositifs médicaux portables comparables, ledit ensemble de données (15) comprenant également des données au sujet de défaillances antérieures quelconques des dispositifs médicaux portables comparables.
